(19) Europäisches Patentamt
European Patent Office
Office européen des brevets

(11) EP 3 171 806 B1

## (12) EUROPEAN PATENT SPECIFICATION

(45) Date of publication and mention
of the grant of the patent:
01.05.2019  Bulletin 2019/18

(21) Application number: 15741219.8

(22) Date of filing: 24.07.2015

(51) Int Cl.:
A61B 18/20 (2006.01)    A61B 18/00 (2006.01)

(86) International application number:
PCT/EP2015/066982

(87) International publication number:
WO 2016/012584 (28.01.2016 Gazette 2016/04)

## (54) A DEVICE FOR CUTTING HAIR

VORRICHTUNG ZUM SCHNEIDEN VON HAAR

DISPOSITIF POUR COUPE DE CHEVEUX

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority:  25.07.2014  EP 14178615

(43) Date of publication of application:
31.05.2017  Bulletin 2017/22

(73) Proprietor: Koninklijke Philips N.V.
5656 AE Eindhoven (NL)

(72) Inventors:
• MOESKOPS, Bastiaan Wilhelmus Maria
5656 AE Eindhoven (NL)
• JOHNSON, Mark Thomas
5656 AE Eindhoven (NL)
• COOMBS, James Howard
5656 AE Eindhoven (NL)
• VERHAGEN, Rieko
5656 AE Eindhoven (NL)
• CIUHU, Calina
5656 AE Eindhoven (NL)
• JUTTE, Petrus Theodorus
5656 AE Eindhoven (NL)

(74) Representative: van Oudheusden-Perset, Laure E.
Philips International B.V.
Intellectual Property & Standards
High Tech Campus 5
5656 AE Eindhoven (NL)

(56) References cited:
WO-A1-92/16338        WO-A1-95/33600
WO-A1-2013/175355     WO-A1-2014/020512
WO-A1-2014/139968     GB-A- 2 495 248
US-A- 5 993 440

## Description

FIELD OF THE INVENTION

The present invention relates to a device for cutting hair using a laser beam. The present invention also relates to a method of cutting hair, and a computer program comprising instructions which, when executed by at least one processor, cause the method to be performed.

BACKGROUND OF THE INVENTION

It is known to use a laser beam to sever hair as an alternative to an arrangement of mechanical cutting blades. Hair exposed to a laser beam will absorb energy from the laser beam and the hair will either be severed by vaporisation or by laser induced optical breakdown and a resulting shockwave. A laser beam requires no moving parts and so the problem of cutting elements becoming worn or blunt is eliminated. Moreover, use of a laser beam to sever hair avoids skin irritation caused by the sharp edges of mechanical blades contacting the skin. It is known from WO1992/16 338 and US5,993,440 to provide a device including a laser diode and reflective elements that direct a laser beam across a cutting zone so that the beam is substantially parallel to and spaced from the surface of the user's skin during use. In this way, as the device is moved across the skin, hairs that enter the cutting zone are exposed to the laser beam and severed at the point of interaction between the laser beam and the hair. WO 2013/175355 A1 discloses a laser shaver comprising an optical system which directs a laser beam parallel to the skin to cut hairs as the shaver is moved over the skin.

Shaving performance is typically measured by two criteria - closeness of shave and irritation of the skin. The cutting height is the distance between the surface of the skin and the point at which hairs are cut. A good performing shaver should minimise the cutting height and therefore minimise the remaining hair length by cutting the hairs as close as possible to the skin. However, positioning a laser beam close to the skin may cause skin irritation if heat and energy from the laser is incident on the skin. It is therefore necessary to protect the skin from a laser beam to avoid causing damage or irritation to the skin.

SUMMARY OF THE INVENTION

It is an object of the invention to provide a device for cutting hair using a laser beam and/or a method of cutting hair which substantially alleviates or overcomes the problems mentioned above.

The invention is defined by the independent claims; the dependent claims define advantageous embodiments.

According to one aspect of the present invention, there is provided a device for cutting hair comprising a skin contacting face that is arranged to be placed against a surface of the skin of a user during use, an optical system configured to direct a cutting laser beam across a cutting zone parallel to and spaced from said skin contacting face to cut hairs extending into the cutting zone, a detector configured to generate information indicative of the presence of one or more objects representative of hair and/or skin in the cutting zone, and a controller configured to determine the presence of one or more objects representative of hair and/or skin in the cutting zone and to adjust one or more characteristics of the optical system in dependence on the information generated by the detector, characterised the controller is configured to generate information indicative of the distance between the one or more objects representative of hair and skin in the cutting zone and the cutting laser beam in dependence on information generated by the detector, and to adjust the intensity of the cutting laser beam in dependence on the information generated by the controller.

This means that it is possible to vary the optical properties of the optical system without the provision of any additional components. Therefore, it is possible to maximise the reliability of the optical system and, therefore, the device.

The controller may be configured to reduce the intensity of the cutting laser beam in dependence on the determination of the presence of one or more objects representative of skin in the cutting zone.

Therefore, the device may be simply operated to prevent the cutting laser beam operating at an intensity sufficient to cut a hair from being incident with skin.

The controller may be configured to increase the intensity of the cutting laser beam in dependence on determination of the presence of one or more objects representative of hair in the cutting zone.

With this arrangement it is possible to maximise the efficiency of the device by operating the cutting laser beam at a reduced intensity level when a hair is not detected in the cutting zone.

The controller may additionally be configured to adjust the path of the cutting laser beam.

Therefore, it is possible to compensate for any deviation in the path of the cutting laser beam.

According to another aspect of the present invention, there is provided a device for cutting hair comprising a skin contacting face that is arranged to be placed against a surface of the skin of a user during use, an optical system configured to direct a cutting laser beam across a cutting zone parallel to and spaced from said skin contacting face to cut hairs extending into the cutting zone, a detector configured to generate information indicative of the presence of one or more objects representative of hair and/or skin in the cutting zone, and a controller configured to determine the presence of one or more objects representative of hair and/or skin in the cutting zone and to adjust one or more characteristics of the optical system in dependence on the information generated by the detector, characterised in that the controller is configured to generate information indicative of the distance between the one or more objects representative of hair and skin in the cutting zone and the cutting laser beam in dependence on information generated by the detector, and to adjust the path of the cutting laser beam in dependence on the information generated by the controller and in that the detector comprises an imaging sensor configured to generate information indicative of the presence of one or more objects representative of hair and/or skin received in a path of a light source directed across the cutting zone.

Again, this therefore renders it possible to compensate for any deviation in the path of the cutting laser beam.

With the above arrangements, it is possible to vary one or more characteristics of the optical system in dependence on the detection of hair and/or skin in the cutting zone. Therefore, it is possible to restrict the cutting laser beam from causing damage or irritation to the skin. Furthermore, it is possible to maximise the efficiency of the device.

The detector may comprise an imaging sensor configured to generate information indicative of the presence of one or more objects representative of hair and/or skin received in a path of a light source directed across the cutting zone.

With this arrangement it is possible to directly determine the presence of an object in the cutting zone. Therefore, it is possible to maximise the accuracy of the detector.

The light source may be a laser beam.

The light source may be the cutting laser beam. The light source may be an auxiliary section of the cutting laser beam.

Therefore, it is possible to use the cutting laser beam to also provide the light source for the imaging sensor. This means that the complexity of the device is minimised and so the reliability may be maximised.

The controller may be configured to generate information indicative of the distance between the cutting laser beam and a reference position in dependence on information generated by the detector.

With this arrangement it is possible to compensate for any deviation in the path of the cutting laser beam by determining both the position of the cutting laser beam and objects in the cutting zone.

The detector may comprise a distance sensor configured to generate information indicative of the distance between one or more objects representative of hair and/or skin received in the cutting zone and a reference position.

With this arrangement it is possible to determine when skin protrudes into the cutting zone based on the height of skin and so minimise damage or irritation to the skin.

The controller may be configured to determine the presence of one or more objects representative of hair and skin in the cutting zone, and to disregard the presence of one or more objects representative of hair in the cutting zone when said one or more objects representative of hair in the cutting zone is determined.

With this arrangement, it is possible to accurately determine the presence of skin in the cutting zone by disregarding false positives generated by hair extending from the skin. This helps to produce a closer shave.

The controller may be configured to adjust the optical system to modify the distance between the path of the cutting laser beam and the skin contacting face.

According to another aspect of the present invention, there is provided a method of cutting hair comprising directing a cutting laser beam across a cutting zone parallel to and spaced from said skin contacting face to cut hairs extending into the cutting zone using an optical system, generating information indicative of the presence of one or more objects representative of hair and/or skin in the cutting zone using a detector, determining the presence of one or more objects representative of hair and/or skin in the cutting zone, and adjusting one or more characteristics of the optical system in dependence on the information generated by the detector, and using a controller to generate information indicative of the distance between the one or more objects representative of hair and skin in the cutting zone and the cutting laser beam in dependence on information generated by the detector and to adjust the intensity of the cutting laser beam in dependence on the information generated by the controller.

According to another aspect of the present invention, there is provided a method of cutting hair comprising directing a cutting laser beam across a cutting zone parallel to and spaced from said skin contacting face to cut hairs extending into the cutting zone using an optical system, generating information indicative of the presence of one or more objects representative of hair and/or skin in the cutting zone using a detector, wherein the detector comprises an imaging sensor configured to generate information indicative of the presence of one or more objects representative of hair and/or skin received in a path of a light source directed across the cutting zone, determining the presence of one or more objects representative of hair and/or skin in the cutting zone, and adjusting one or more characteristics of the optical system in dependence on the information generated by the detector, and using a controller to generate information indicative of the distance between the one or more objects representative of hair and skin in the cutting zone and the cutting laser beam in dependence on information generated by the detector and to adjust the path of the cutting laser beam in dependence on the information generated by the controller.

According to another aspect of the present invention, there is provided a computer program comprising instructions which, when executed by at least one processor, cause the methods described above to be performed.

These and other aspects of the invention will be apparent from and elucidated with reference to the embodiments described hereinafter.

BRIEF DESCRIPTION OF THE DRAWINGS

Embodiments of the invention will now be described, by way of example only, with reference to the accompanying drawings, in which:

FIG. 1 shows a view of the cutting end of a device for cutting hair using a laser beam;
FIG. 2 shows a front view schematic diagram of the cutting end of the device of FIG. 1;
FIG. 3 shows a schematic block circuit diagram of the embodiment of the device shown in FIG. 2;
FIG. 4 shows an illustrative view of information indicative of a laser beam profile generated by an imaging sensor of the device of FIG. 1 showing a hair;
FIG. 5 shows an illustrative view of information indicative of a laser beam profile generated by an imaging sensor of the device of FIG. 1 showing a hair and skin;
FIG. 6 shows flow diagram of a routine performed by a controller in the device;
FIG. 7 shows front view schematic diagram of a further embodiment of the cutting end of the device of FIG. 1;
FIG. 8 shows a schematic block circuit diagram of the embodiment of the device shown in FIG. 7;
FIG. 9 shows an illustrative chart of information generated by a distance sensor of the embodiment of the device of FIG. 7;
FIG. 10 shows front view schematic diagram of a different embodiment of the cutting end of the device of FIG. 1;
FIG. 11 shows front view schematic diagram of a different embodiment of the cutting end of the device of FIG. 1; and
FIG. 12 shows a schematic block circuit diagram of the embodiment of the device shown in FIG. 10.

DETAILED DESCRIPTION OF EMBODIMENTS

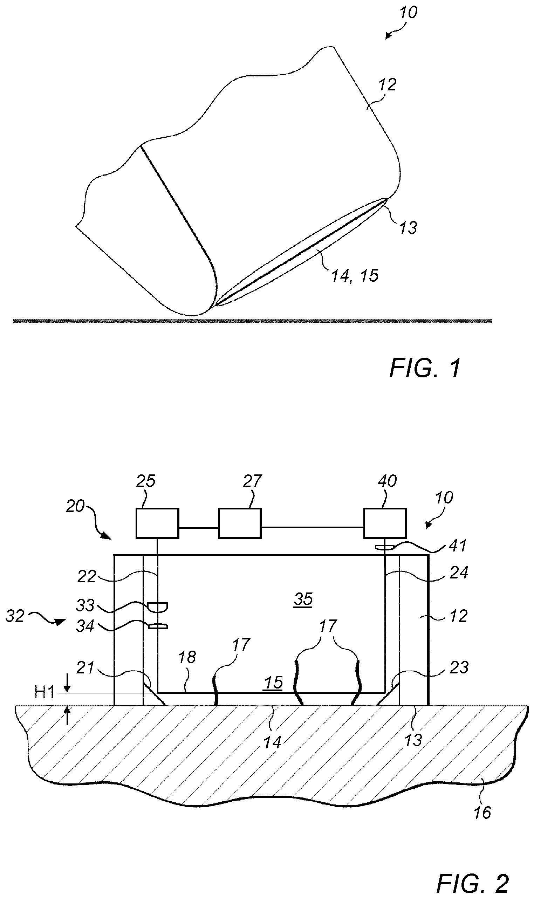

Referring in particular to FIGS. 1 to 3, a device 10 for cutting hair is shown. The device 10 comprises a main body 12 which includes a skin contacting face 13 having an opening 14. As shown in FIG. 2, during use, the skin contacting face 13 of the main body 12 is placed against skin 16 of a user and hairs 17 on the skin 16 protrude into the opening 14 of the main body 12 and into a cutting zone 15 defined therein. Within the main body 12, the device 10 comprises an optical system 20 which directs a cutting laser beam 18 across the cutting zone 15 so that the cutting laser beam 18 is parallel to and spaced from the skin contacting face 13. The cutting laser beam 18 also extends across the opening 14 of the main body 12. In this way, when the skin contacting face 13 of the main body 12 is placed against the skin 16 of the user, the cutting laser beam 18 is substantially parallel to and spaced from the surface of the skin 16 of the user and provides a substantially uniform cutting height across the cutting zone 15. The distance between the surface of the skin 16 and the cutting laser beam 18 in the cutting zone 15, represented as distance H1 in FIG. 2, is the cutting height.

The optical system 20 comprises a first reflective element 21 positioned on one side of the opening 14 and cutting zone 15. The first reflective element 21 is configured to reflect an incident section 22 of the cutting laser beam 18 across the opening 14 at the end of the device 10. That is, the first reflective element 21 is configured to reflect the incident section 22 of the cutting laser beam 18 across the cutting zone 15, such that the cutting laser beam 18 follows a path which is substantially parallel to and spaced from the skin contacting face 13 and opening 14 of the device 10.

On an opposite side of the cutting zone 15 to the first reflective element 21, a second reflective element 23 is positioned to reflect the cutting laser beam 18 away from the cutting zone 15, towards an energy dissipater (not shown) so that the 'used' section 24 of the cutting laser beam 18 is dissipated and can not interact with a part of the user or another part of the device. In the example shown in FIG. 2, the incident section 22 of the cutting laser beam 18 which is incident on the first reflective element 21 is perpendicular to the skin contacting face 13 of the main body 12 and the first reflective element 21 reflects the laser beam through 90 degrees such that it is parallel to the skin contacting face 13 of the main body 12. Similarly, the second reflective element 23 is configured to reflect the cutting laser beam 18 through 90 degrees and perpendicularly away from the cutting zone 15. However, it will be appreciated that the first and second reflective elements 21, 23 may be orientated differently or have different reflective angles, depending on the position and orientation of other parts of the optical system, such as a laser beam generator 25 and the energy dissipater (not shown). Furthermore, it will be appreciated that the first and second reflective elements 21, 23 may not be located at a side of the cutting zone 15. They may alternatively be located anywhere within the cutting zone 15, depending on the position, orientation and configuration of the other components of the optical system 20. The first and/or second reflective elements 21, 23 may be omitted. However, the cutting laser beam 18 within the cutting zone 15 should remain parallel to the skin contacting face 13 and opening 14 of the main body 12 so that the distance between the skin contacting face 13 and the cutting laser beam 18 is constant across the cutting zone 15. Any reflective elements described herein may comprise a mirror or a prism or any other optically reflective surface.

The optical system 20 of the device 10 further comprises the laser beam generator 25, such as a diode.

As shown in FIG. 2, the optical system 20 includes a lens arrangement 32. The lens arrangement 32 comprises one or more lens elements. In the present embodiment, the lens arrangement 32 includes a collimating lens 33 and a focus lens 34. The laser beam emitted by the laser beam generator 25 is directed towards the collimating lens 33. The collimating lens 33 reduces or eliminates the divergence of the laser beam emitted by the laser beam generator 25. The collimated section or path of the laser beam is then focused by the focus lens 34 to cause the laser beam to converge.

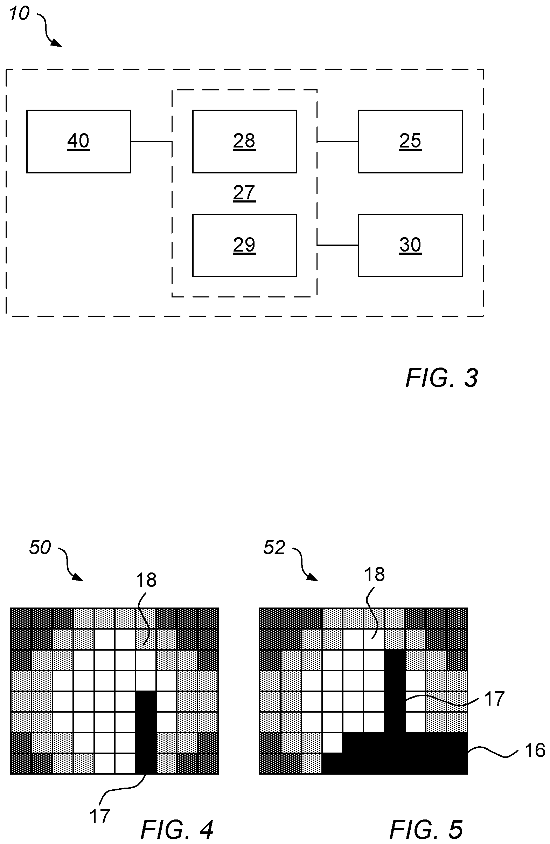

The device 10 also comprises a controller 27. The controller 27 is configured to control operation of the laser beam generator 25, and so control operation of the cutting laser beam 18. Referring to FIG. 3, the controller 27 comprises a processor 28 and a memory 29. The controller 27 is operable to operate the optical system 20.

The processor 28 may take any suitable form. For instance, the processor 28 may be or include a microcontroller, plural microcontrollers, circuitry, a single processor, or plural processors. The controller 27 may be formed of one or multiple modules.

The memory 29 takes any suitable form. The memory 29 may include a non-volatile memory and/or RAM. The non-volatile memory may include read only memory (ROM), a hard disk drive (HDD) or a solid state drive (SSD). The memory stores, amongst other things, an operating system. The memory may be disposed remotely. The RAM is used by the processor 28 for the temporary storage of data. The operating system may contain code which, when executed by the controller 27, controls operation of each of the hardware components of the device 10, or alternatively a system including the device 10. The controller 27 may be able to cause one or more objects, such as one or more profiles, to be stored remotely or locally by the memory 29. The controller 27 may be able to refer to one or more objects, such as one or more profiles, stored by the non-volatile memory and upload the one or more stored objects to the RAM.

The controller 27 is operable to operate the device 10 in response to an input, for example a user input 30. The controller 27 is configured to operate the optical system 20.

The user input 30 comprises some form of user interface. Optionally, the device 10 includes controls and/or displays for adjusting some operating characteristic of the device, such as the power. The user input 30 allows a user to operate the device 10, for example to turn the device 10 on and off. The user input 30 may, for example, be a button, touch screen or switch.

Other components that the device 10 may comprise, which are not shown, include other optical components such as a filter or windows to limit the passage of detritus in the device 10. Other components necessary for the operation of the device may also be located within the main body 12, such as a battery or a connection to an external power cable (not shown). Moreover, the main body 12 of the device 10 may also comprise a handle and any switches, buttons or other controls and displays necessary to operate the device, which may form the user input 30.

The cutting zone 15 is formed in a chamber 35 in the main body 2. The opening 14 to the cutting zone 15 is defined by an opening to the chamber 35.

The device 10 is provided with an imaging sensor 40. The imaging sensor 40 is an electronic sensor which is configured to generate information indicative of one or more optical properties of a light source directed at the imaging sensor 40. The imaging sensor 40 is configured to generate information indicative of the light source intensity profile at the imaging sensor 40. In the present embodiment the light source is the cutting laser beam 18 when the optical system 20 is operated. That information is provided to the controller 27. The controller 27 may then control operation of one or more components of the optical system 20 in dependence on the information provided by the imaging sensor 40.

The imaging sensor 40 is an optical imaging sensor. The imaging sensor 40 in the present embodiment is a multi-element optical imaging detector. The optical imaging sensor may be a photodiode array. The imaging sensor 40 is disposed at the end of the optical path of the cutting laser beam 18. In the present embodiment, the imaging sensor 40 is at the energy dissipater (not shown). However, it will be understood that alternative sensor arrangements may be used.

The imaging sensor 40 is configured to detect one or more optical properties of the cutting laser beam 18. A detector lens 41 is disposed on the cutting laser beam path prior to the imaging sensor 40 to adjust the dimensions of the cutting laser beam 18 to suit the imaging sensor 40. For example, the detector lens 41 may be configured to ensure that the dimensions of the cutting laser beam 18 at the imaging sensor 40 correspond to the resolution of the imaging sensor 40. The detector lens 41 may be omitted.

The imaging sensor 40 intersects the path of the cutting laser beam 18. The imaging sensor 40 is configured to determine the intensity of the cutting laser beam 18 at the imaging sensor 40. In the present arrangement the imaging sensor 40 is at the end of the path of the cutting laser beam 18.

The imaging sensor 40 is configured to generate information indicative of the intensity of the cutting laser beam 18. The imaging sensor 40 is configured to generate information indicative of the intensity of different regions of the cutting laser beam 18. The imaging sensor 40 has a multi-element array in the present embodiment, and so each element is able to provide information indicative of the intensity of the region of the cutting laser beam 18 intersecting that element. The imaging sensor 40 is able to generate a profile providing information indicative of the intensity of different regions of the light source intersecting the imaging sensor 40. Such a profile is illustratively shown in FIG. 4. In FIG. 4 a profile 50 indicative of hair 17 only being received in the path of the cutting laser beam 18, acting as the light source, is shown. In FIG. 5 a profile 52 indicative of hair 17 and skin 16 being received in the path of the cutting laser beam 18, acting as the light source is shown.

The controller 27 is configured to refer to the information generated by the imaging sensor 40 indicative of the intensity profile of the cutting laser beam 18 at the imaging sensor 40. Based on the information provided to the controller 27, the controller 27 is configured to determine the presence of one or more objects received in the path of the cutting laser beam 18. Any object received in the path of a light source directed at the imaging sensor 40 will act to restrict light from hitting the imaging sensor 40 on at least part of the imaging sensor 40. That is, the or each object will create a shadow. Such a shadow of an object is shown on the intensity profile in FIG. 4. Therefore, the controller 27 is able to determine whether an object is received in the path of the light source, and to identify the type of object received in the path of the light source.

The controller 27 is configured to identify the type of object received in the path of the cutting laser beam 18 based on the information generated by the imaging sensor 40. The controller 27 is configured to refer to one or more frames output by the imaging sensor 40 providing a profile. Based on the information generated by the imaging sensor 40, the controller 27 is configured to use object recognition to determine the type of object. This may be conducted by comparison with a reference profile stored by the memory 29. Such object recognition is known and so a detailed description will be omitted herein.

Based on the object recognition provided by the controller 27, the controller 27 is configured to determine the type of object or objects received in the path of the cutting laser beam 18. The controller 27 is configured to identify when an object is received in the path of the cutting laser beam 18. The controller 27 is configured to adjust the intensity of the cutting laser beam 18 in dependence on the determined identification of an object in the path of the cutting laser beam 18 based on the information generated by the imaging sensor 40.

During use, hair 17 is received in the path of the cutting laser beam 18. It will be understood that the intensity of the cutting laser beam 18 will vary across its cross-sectional profile, and so will have a lower intensity towards its outer edge. Therefore, it is possible for an object to be identified by imaging sensor 40 without the cutting laser beam 18 imparting a cutting action on it. A cutting edge of the cutting laser beam 18 is defined as a periphery of the cutting laser beam 18 having a laser intensity sufficient to provide a cutting action on a desired hair type. In the present embodiment, the cutting edge of the cutting laser beam 18 is defined as a periphery of the cutting laser beam 18 containing 99% of the laser intensity, however it will be understood that in alternative embodiments the percentage may differ.

Skin 16 may also be received in the path of the cutting laser beam 18 (refer to FIG. 5). The distance between the cutting laser beam 18 and the skin 16 at the cutting zone 15 may vary in response to changes in the height of the skin 16 of a user within the cutting zone 15, for example due to skin height variations and the skin doming in the cutting zone 15. Therefore, the skin 16 may move closer to, and further away from, the cutting laser beam 18 during use of the device 10. The controller 27 is configured to determine when an object is received in the path of the cutting laser beam 18 by reference to the information generated by the imaging sensor 40.

The controller 27 is configured to identify the type of object received in the path of the cutting laser beam 18 based on the information generated by the imaging sensor 40. The controller 27 may refer to a reference profile stored by the memory 29. When the controller 27 identifies that an object is skin, the controller 27 is configured to reduce the intensity of the cutting laser beam 18, for example the power or the energy of the laser beam. Therefore, the amount of laser energy imparted on the skin is minimised or prevented. In this way, variations in the skin height, such as skin contours, can be accommodated whilst minimising skin irritation. In the present embodiment, the controller 27 is configured to determine the cutting height, that is the distance between the cutting edge of the cutting laser beam 18 and the skin 16 by disregarding information indicative of hair 17 in the cutting zone 15. This means that an accurate determination of the cutting height may be determined based on the information indicative of skin only.

In the present embodiment, the controller 27 operates the laser beam generator 25 to produce the cutting laser beam 18 at two different laser beam intensity levels. For a first laser beam intensity level, the desired intensity of the cutting laser beam 18 is less than the intensity required to cause damage to skin. That is, a skin safe, detection level, of intensity. For a second laser beam intensity level, the desired intensity of the cutting laser beam 18 is equal to or greater than the intensity required to cause a cutting action on hair. The laser beam intensity levels may be stored by the memory 28 and referred to by the controller 27.

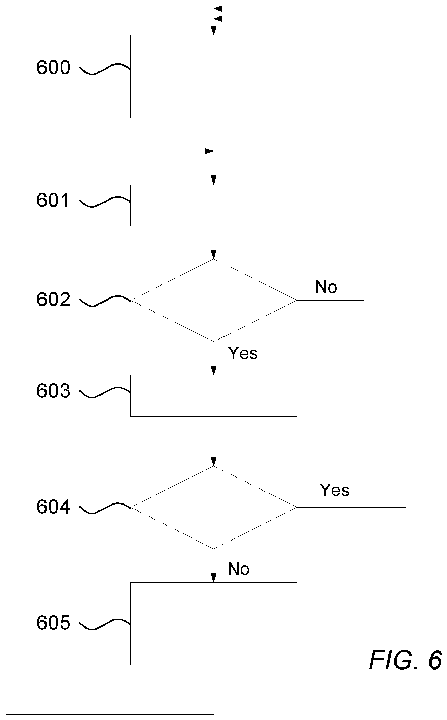

FIG. 6 shows a process performed by the controller 27 to operate the cutting laser beam. At step 600 the cutting laser beam 18 is operated at the first laser beam intensity level.

At step 601, information generated by the imaging sensor 40 indicative of the presence of one or more objects in the path of the cutting laser beam 18, acting as the light source, is referred to by the controller 27. The presence of one or more objects is tested at step 602. If the controller 27 determines that no object is present in the path of the cutting laser beam 18, acting as the light source, then the routine is repeated.

If the controller 27 determines that an object is present in the path of the cutting laser beam 18, acting as the light source, then the controller 27 determines the type of object based on an object recognition process at step 603. Determination of the type of object is tested at step 604. If the presence of skin is identified by the controller 27 based on the generated information then the process is repeated and operation of the cutting laser beam 18 is maintained at the first laser beam intensity level. If the presence of skin is not identified by the controller 27 then the process is repeated until the presence of skin is not determined. In the event that skin is not identified, and so the presence of hair is identified by the controller, then the cutting laser beam is operated at the second laser beam intensity level at step 605. The process is repeated until an object is not detected or the presence of skin is determined, in which case the cutting laser beam is operated at the first laser beam intensity level.

Information indicative of the desired intensity for a given cutting height is stored in the memory 29 in a reference profile. The controller 27 is configured to refer to the reference profile of the desired intensity in response to information indicative of the distance between the lower edge of the cutting laser beam 18 and the skin 16 at the cutting zone 15. The reference profile may be stored in a look-up table. The reference profile may be stored by the memory 29. In such an arrangement, the controller 27 is configured to refer to the memory 29 to access the reference profile. In one embodiment, the reference profile is stored by the RAM. The reference profile provides information indicative of the desired intensity.

It will be understood that the controller 27 is configured to select the reference profile from two or more reference profiles. That is, each reference profile may provide a desired operating intensity based on the determined cutting height. The two or more reference profiles may be stored in the memory 29. Alternatively, the reference profile may include two or more desired intensity levels from which the controller 27 is able to select based on the determined cutting height. The user may be able to select a desired closeness of shave. For example, in an embodiment in which the reference profile includes two or more desired intensity levels from which the controller 27 is able to select based on the determined cutting height, the user may be able to select from two or more reference profiles each of which has a different intensity level for a given cutting height.

When the device 10 is operated, the controller 27 is configured to operate the laser beam generator 25 to adjust the intensity of the cutting laser beam 18 in dependence on the cutting height determined by the controller 27 by reference to the information generated by the imaging sensor 40.

In one embodiment, the controller 27 is operable to adjust the intensity of the cutting laser beam 18 in dependence on a minimum threshold value of the cutting height, as determined by the information generated by the imaging sensor 40. In such an embodiment, the controller 27 is operable to operate the laser beam generator 25 so that the cutting laser beam 18 operates at a first intensity level when the cutting height is determined to be less than or equal to the minimum threshold value.

and the controller 27 is operable to operate the laser beam generator 25 so that the cutting laser beam 18 operates at a second intensity level when the cutting height is greater than the minimum threshold value, The minimum threshold value is stored by the one or more reference profiles. With such an arrangement the controller 27 is configured to enact a step change in the intensity level of the cutting laser beam 18.

In an alternative embodiment, the reference profile may include one or more threshold values for the cutting height as determined by the controller 27 based on information provided by the imaging sensor 40. In such an embodiment, each threshold value has a corresponding intensity level. The two or more threshold values and corresponding intensity levels may be stored by the reference profile. In another alternative, the controller 27 is configured to determine the desired intensity level for the cutting laser beam 18 by operating the laser beam generator 25 to constantly vary the intensity level based on information provided by the imaging sensor 40.

Although in the above embodiment the controller 27 is configured to initially operate the cutting laser beam 18 at a first intensity level, that is a skin-safe, detection, level, when the presence of an object is not detected in the path of the cutting laser beam 18, it will be understood that, in an alternative embodiment, the cutting laser beam 18 is configured to be initially operated at the second laser beam intensity level, which is at a level equal to or greater than an intensity needed to cause a cutting action on hair 17.

In such an embodiment, during use the cutting laser beam 18 is initially operated at the second laser beam intensity level. If the controller 27 determines that a hair only is in the path of the cutting laser beam 18 in view of the information provided by the imaging sensor 40, then the controller 27 is configured to maintain the intensity level of the cutting laser beam 18 at equal to or greater than an intensity required to cause a cutting action on hair 17. If the controller 27 determines that skin 16 is in the path of the cutting laser beam 18 in view of the information provided by the imaging sensor 40, then the controller 27 is configured to reduce the intensity level of the cutting laser beam 18 to operate at the first laser beam intensity level.

In the above described embodiments, the cutting laser beam 18 is incident with the imaging sensor 40 and the intensity profile 50, 52 is used to determine the presence of one or more objects in the path of the cutting laser beam 18. However, in a further embodiment it will be understood that the relative position of the cutting laser beam 18 may also be determined. In such an embodiment, the cutting laser beam 18 is incident with the imaging sensor 40 and the controller 27 is configured to determine the path of the cutting laser beam 18 based on the information generated by the imaging sensor 40. That is, the controller 27 is configured to also determine the position of the cutting edge of the cutting laser beam 18 on the imaging sensor 40. As the position of the cutting edge of the cutting laser beam 18 on the imaging sensor 40 is determined, as well as the position of one or more objects, it will be appreciated that the controller 27 is able to compensate for any movement or discrepancies in the position and, therefore, path of the cutting laser beam 18 in the cutting zone 15.

Due to the controller 27 being able to determine both the position of the cutting laser beam 18 in the cutting zone 15 and the skin 16 at the cutting zone 15, the controller 27 is able to accurately determine the distance between the cutting edge of the cutting laser beam 18 and the skin 16. This also enables any variations during use or inbetween use to be taken into account. With such an arrangement, optical element alignment variations may be taken into account. Such optical element alignment variations, for example, may occur due to a user dropping the device 10.

In the above described embodiment, the cutting laser beam 18 acts as the light source acting on the imaging sensor 40. However, it will be understood that alternative embodiments are envisaged. For example, in one embodiment the cutting laser beam is split into a primary cutting beam 18 and an auxiliary detection beam (not shown). Alternatively, the auxiliary detection beam is a separate light source, for example, an independent laser beam. In such embodiments, the primary cutting beam and the auxiliary detection beam follow paths across the cutting zone 15. The auxiliary detection beam extends parallel to the primary cutting beam 18. The auxiliary detection beam is incident with the imaging sensor 40. With this arrangement, the intensity of the auxiliary detection beam may be continuously maintained at a skin-safe intensity level throughout operation of the device 10.

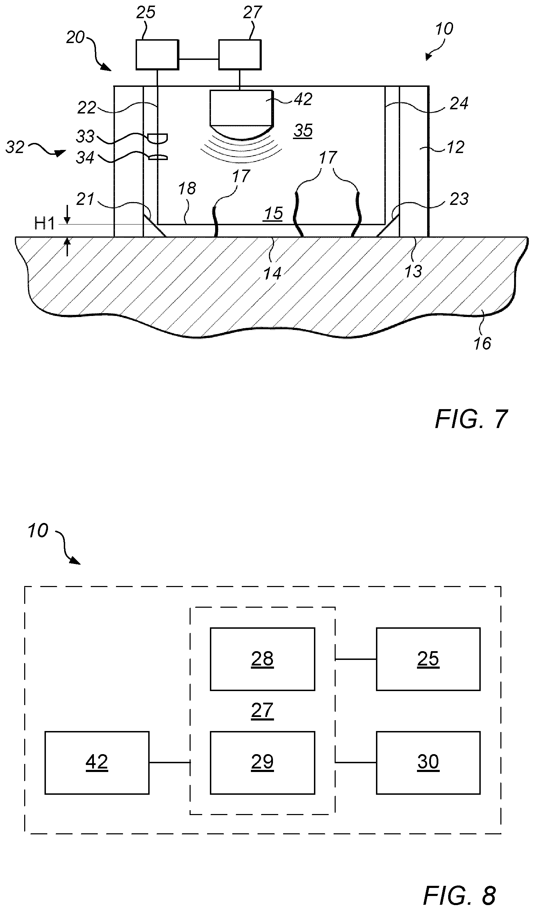

Although in the above described embodiments the imaging sensor 40 is configured to act as a skin presence detector, it will be understood that in an alternative embodiment a different type of skin presence detector may be used. Referring now to FIGS. 7 and 8, an alternative embodiment of a device 10 for cutting hair is shown. The device 10 shown in FIGS. 7 and 8 is generally the same as the devices described above, and so a detailed description will be omitted. Reference numerals will be retained and refer to features and components described in detail above. However, in this embodiment the skin presence detector is a distance sensor 42.

The distance sensor 42 is an electronic sensor which is configured to generate information indicative of the distance between skin 16 at the cutting zone 15 and a reference position when the device 1 is positioned against the skin 16. This distance is the skin height. That information is provided to the controller 27 which controls operation of the laser generator 25. The distance sensor 42 is an optical sensor, such as a confocal lens which uses optical measuring techniques and does not need to contact the skin 16 to measure the distance between the surface of the skin 16 and the reference position on the device 10. The distance sensor 42 may be configured to generate information indicative of the position of the skin 16 by, for example, triangulation measurement, scattered light measurement and/or shadow measurement. In the present embodiment, the reference position is based on the skin contacting face 13. Therefore, the provided information indicative of the distance between the skin 16 at the cutting zone 15 and said reference position is the perpendicular distance between the skin at the cutting zone 15, as determined by the distance sensor 42, and the plane of the skin contacting face 13. However, it will be understood that an alternative reference position may be used. For example, the reference position may be a neutral calibrated position of the cutting laser beam 18. The cutting height, that is the the distance between the surface of the skin 16 and the cutting laser beam 18 in the cutting zone 15, may be calculated from the reference position.

The distance sensor 42 is disposed in the cutting zone 15. That is, the distance sensor 42 is disposed in the chamber 35 in the main body 12. The distance sensor 42 is a noncontact skin distance sensor. That is, the distance sensor 42 is configured to measure the distance between skin 16 at the cutting zone 15 and the reference position when the device 1 is positioned against the skin 16. The distance sensor 42 may be a reflective or transmissive optical sensor. The distance sensor 42 may use one or more wavelengths of light in the visible and/or near infra-red radiation regions. However, it will be understood that alternative sensor arrangements may be used. The distance sensor 42 is configured to determine the distance between skin 16 at the cutting zone 15 and the reference position along the propagation direction of the cutting laser beam 18. That is, along the length of the cutting zone 15.

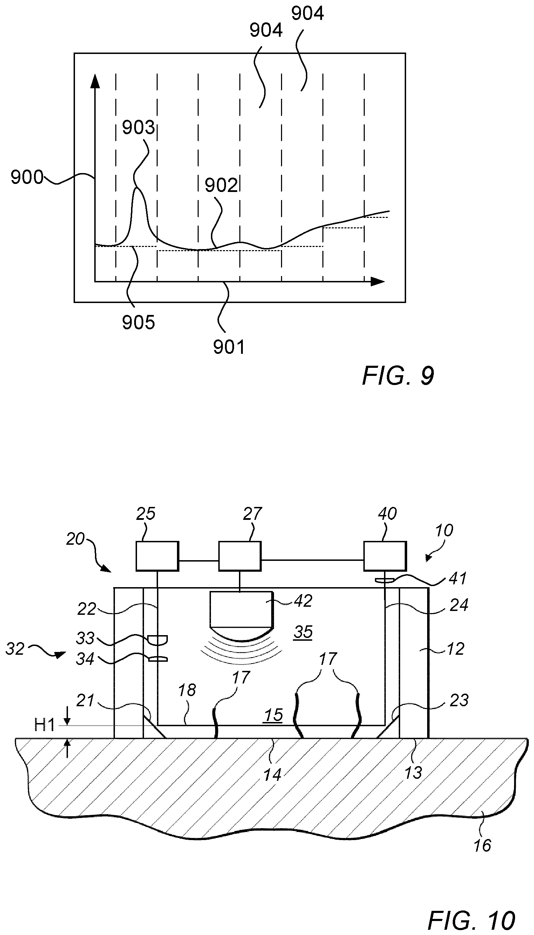

The distance sensor 42 is configured to determine information indicative of the presence and distance from an object such as hair and/or skin in the cutting zone 15 across the length of the cutting zone 14 in the propagation direction of the incident section 22 of the cutting laser beam 18. Referring to FIG. 9, the propagation direction is represented by axis 901. The height of objects is represented by axis 900. The distance sensor 42 is configured to provide information indicative of the height of objects along the propagation direction of the incident section 22 of the cutting laser beam 18 as shown in FIG. 9 by line 902. It will be understood that the peak 903 is representative of a hair 17 in the cutting zone 15, with the remainder of the line 902 being representative of skin 16. It will be understood that the cutting device 10 is configured to be moved along skin 16 in a direction perpendicular to the propagation direction of the incident section 22 of the cutting laser beam 18.

The controller 27 is configured to refer to information indicative of a height of one or more objects, such as hair 17 and skin 16, in the cutting zone 15 generated by the distance sensor 42. The controller 27 is configured to divide the information into discrete bins 904 representative of a section of the cutting zone 15 along the propagation direction of the incident section 22 of the cutting laser beam 18. That is, the cutting zone 15 is nominally divided into an array of discrete bins 904. Each bin 903 is configured to represent a distance greater than the width of hair.

Based on the information generated by the distance sensor 42 for each bin 904, a minimum height, that is a minimum distance of protrusion into the cutting zone 15 of the or each object is provided for each bin 904. This minimum height is represented by the dotted line 905 for each bin 904 in FIG. 9. The controller 27 is configured to determine the position of skin for each bin 904 based on the minimum height. Therefore, the peak 903 representative of hair will be disregarded. Based on the determined minimum height for each bin 904, the controller 27 is configured to determine the cutting height. The cutting height is determined as the greatest minimum height of the skin across the array of bins 904.

Information indicative of the desired intensity for a given cutting height is stored in the memory 29 in a reference profile. The controller 27 is configured to refer to the reference profile of the desired intensity in response to information indicative of the distance between the lower edge of the cutting laser beam 18 and the skin 16 at the cutting zone 15 as determined by reference to the minimum height for each bin 904. Therefore, false positives based on hair 17 may be disregarded. The reference profile may be stored in a look-up table. The reference profile may be stored by the memory 29. In such an arrangement, the controller 27 is configured to refer to the memory 29 to access the reference profile. In one embodiment, the reference profile is stored by the RAM. The reference profile provides information indicative of the desired intensity.

It will be understood that the controller 27 may be configured to select the reference profile from two or more reference profiles. That is, each reference profile may provide a desired operating intensity based on the determined cutting height. The two or more reference profiles may be stored in the memory 29. Alternatively, the reference profile may include two or more desired intensity levels from which the controller 27 is able to select based on the determined cutting height. The user may be able to select a desired closeness of shave. For example, in an embodiment in which the reference profile includes two or more desired intensity levels from which the controller 27 is able to select based on the determined cutting height, the user may be able to select from two or more reference profiles each of which has a different intensity level for a given cutting height.

When the device 10 is operated, the controller 27 is configured to operate the laser beam generator 25 to adjust the intensity of the cutting laser beam 18 in dependence on the cutting height determined by the controller 27 by reference to the information generated by the distance sensor 42 for each bin 904.

In one embodiment, the controller 27 is operable to adjust the intensity of the cutting laser beam 18 in dependence on a minimum threshold value of the cutting height, as determined by the information generated by the distance sensor 42. In such an embodiment, the controller 27 is operable to operate the laser beam generator 25 so that the cutting laser beam 18 operates at a second laser beam intensity level when the cutting height is greater than the minimum threshold value, and the controller 27 is operable to operate the laser beam generator 25 so that the cutting laser beam 18 operates at a first laser beam intensity level when the cutting height is determined to be less than or equal to the minimum threshold value. The minimum threshold value is stored by the one or more reference profiles. With such an arrangement the controller 27 is configured to enact a step change in the intensity level of the cutting laser beam 18.

In an alternative embodiment, the reference profile may include one or more threshold values for the cutting height as determined by the controller 27 based on information provided by the distance sensor 42. In such an embodiment, each threshold value has a corresponding intensity level. The two or more threshold values and corresponding intensity levels may be stored by the reference profile. In another alternative, the controller 27 is configured to determine the desired intensity level for the cutting laser beam 18 by operating the laser beam generator 25 to constantly vary the intensity level based on information provided by the distance sensor 42.

Although in the present arrangement the distance sensor 42 is disposed in the cutting zone 15 and is configured to detect the skin 16 positioned at the opening 14 to the cutting zone 15, it will be understood that in an alternative embodiment the distance sensor 42 may be disposed outside the cutting zone 15. For example, the distance sensor 42 may be disposed to detect skin at a sensor opening (not shown) adjacent to the opening 14 to the cutting zone 15. An advantage of the distance sensor 42 being disposed in the cutting zone 15 is that it is more accurately able to determine the skin height at the cutting zone 15.

Although in the above described embodiments, one of an imaging sensor 40 or a distance sensor 42 is used, it will be understood that in an alternative embodiment, the device 10 is provided with both an imaging sensor 40 and a distance sensor 42. Such an embodiment is shown in FIGS. 9 and 10. The arrangement of the imaging sensor 40 and the distance sensor 42 is generally the same as described above and so a detailed description will be omitted. In this embodiment, the controller 27 is configured to determine the presence of one or more objects representative of hair and/or skin in the cutting zone and to adjust operation of the cutting laser beam 18 in dependence on the information generated by the imaging sensor 40 and the distance sensor 42. With such an embodiment, the accuracy of determining the cutting height may be maximised by minimising the number of false positives provided by hair. That is, incorrect cutting heights determined by the controller 27 in view of hair 17 on skin 16.

It has been found that a hair 17 extending along the surface of skin 16 parallel to the propagation direction of the incident section 22 of the cutting laser beam 18 may provide an incorrect cutting height, that is a false positive, for an embodiment in which the detector used is the imaging sensor 40. In such an embodiment, it has been found that a hair extending substantially parallel to the skin along the propagation direction will generate a shadow across the width of the imaging sensor 40. Therefore, the controller 27 may incorrectly determine that the shadow is representative of skin 16, such that the cutting height is incorrectly determined. However, in such an embodiment, a hair 17 extending along the surface of skin 16 perpendicular to the propagation direction of the incident section 22 of the cutting laser beam 18 will not cause this issue.

Similarly, it has been found that a hair 17 extending along the surface of skin 16 in a direction perpendicular to the propagation direction of the incident section 22 of the cutting laser beam 18 may provide an incorrect cutting height, that is a false positive, for an embodiment in which the detector used is the distance sensor 42. In such an embodiment, it has been found that a hair extending substantially parallel to the skin but perpendicular to the propagation direction will generate extend across the full width of at least one bin 904. Therefore, the controller 27 may incorrectly determine that the hair is representative of a minimum height of the bin 904, such that the cutting height is incorrectly determined. However, in such an embodiment, a hair 17 extending along the surface of skin 16 parallel to the propagation direction of the incident section 22 of the cutting laser beam 18 will not cause this issue because the hair 17 will not extend across the full width of a bin 904. Therefore, in an embodiment having a detector with both the imaging sensor 40 and the distance sensor 42 it is possible for the controller to determine the cutting height based on information generated by both the imaging sensor 40 and the distance sensor 42. This means that it is possible to minimise the number of incorrect cutting heights.

Although in the above described embodiments the one or more characteristics of the optical system that the controller 27 is configured to adjust is the intensity of the cutting laser beam 18, it will be understood that, in alternative embodiments, one or more other characteristics of the optical system may be adjusted. The one or more other characteristics of the optical system may be adjusted together adjustment of the intensity of the cutting laser beam 18, or as an alternative to adjustment of the intensity of the cutting laser beam 18.

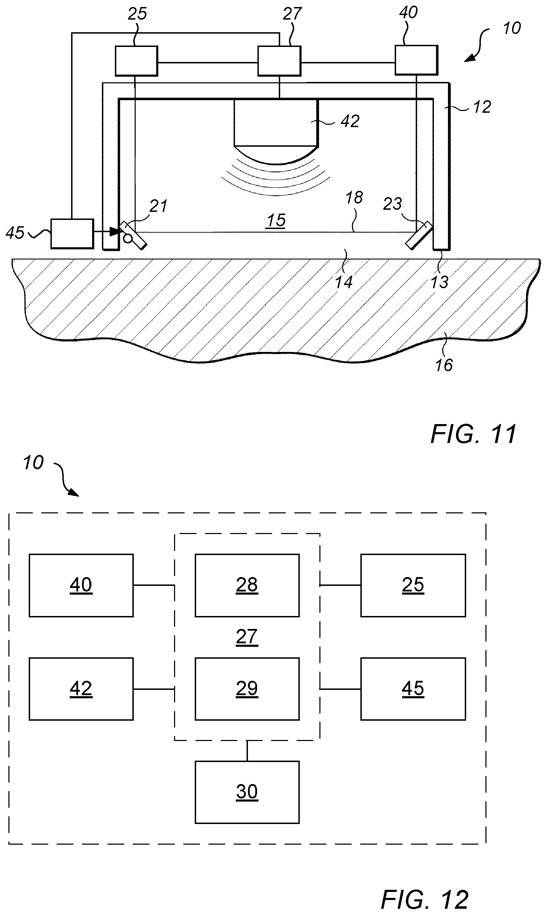

Referring now to FIGS. 11 and 12, another embodiment of the device 10 is shown. The arrangement of this embodiment of the device 10 as shown in FIGS. 11 and 12 is generally the same as the embodiments described above, and so a detailed description will be omitted herein. However, this embodiment of the device 10 further comprises an actuator 45 which is configured to act on one or more optical components of the optical system 20. Reference numerals will be retained and refer to features and components described in detail above.

In the embodiment of the device 10 shown in FIGS. 11 and 12, the actuator 45 is configured to act on the first reflective element 21. The actuator 45 is configured to cause the first reflective element 21 to move. The actuator 45 is arranged to cause rotation of the first reflective element 21. The first reflective element 21 is configured to rotate about a rotational axis which is perpendicular to the propagation direction of the incident section 22 of the cutting laser beam 18. The first reflective element 21 is positioned to reflect the incident section 22 of the cutting laser beam 18 across the cutting zone 15, as previously explained.

The actuator 45 is an electronic actuator, for example a voice coil actuator, spindle motor with gear or a piezo electrical translator. The actuator 45 acts on the first reflective element 21 in response to commands from the controller 27. The controller 27 is configured to operate the actuator 45 to control the propagation angle of the cutting laser beam 18 across the cutting zone 15. That is, movement of the first reflective element 21 allows the path of the cutting laser beam 18 across the cutting zone 15 to be adjusted, and therefore the cutting height.

The controller 27 is configured to operate the actuator 45 to move the first reflective element 21. Therefore, the path of the cutting laser beam 18 is varied in a direction perpendicular to the propagation direction of the cutting laser beam 18 in the cutting zone 15. The controller 27 is configured to refer to one or more of information indicative of the presence of one or more objects representative of hair 17 and/or skin 16 in the cutting zone 15 generated by the imaging sensor 40 and/or distance sensor 42. Therefore, the controller 27 is able to adjust the path of the cutting laser beam 18 to prevent the cutting laser beam 18 from being incident on the skin 16.

In the embodiment described above in which the actuator 45 is configured to act on one or more of the optical components of the optical system 20, the movement that the actuator 45 is configured to cause is rotation. In particular, in the embodiments described herein with reference to FIGS. 11 and 12, the actuator 45 is configured to act on the first reflective element 21 to cause rotation of the first reflective element 21. However, it will be understood that alternative arrangements are envisaged.

In embodiments, it will be understood that the actuator 45 may be configured to act on one or more of the optical components of the optical system 20 to cause translation of the one or more optical components of the optical system 20. For example, in one embodiment the actuator 45 is configured to act on the first reflective element 21 to cause a translational movement of the first reflective element 21. The translational movement may be in a direction towards and/or away from the laser beam generator 25; that is perpendicular to the propagation direction of the cutting laser beam 18 across the cutting zone 15. It will be understood that the second reflective element 23 may move together with the first reflective element 21, or be dimensioned to allow for movement of the first reflective element 21.

In an alternative embodiment, the translational movement of the first reflective element 21 may be in a direction towards and away from the second reflective element 23; that is parallel to the path of the cutting laser beam 18 across the cutting zone 15.

The actuator 45 may alternatively, or also, act on the laser beam generator 25 to cause translation of the laser beam generator 25 itself. In such an embodiment, the actuator 45 is configured to act on the laser beam generator 25 to cause a translational movement parallel to the propagation direction of the cutting laser beam 18 across the cutting zone 15.

The actuator 45 may alternatively, or also, act on the lens arrangement 32. For example, the actuator 45 may act on the focus lens 34. The actuator may cause a translational movement of the focus lens 34 in a direction perpendicular to the optical axis of the cutting laser beam 18 through the lens arrangement 32. For example, if the decentering of the collimating lens 33 with respect to the laser is defined as $\Delta$, the required translation, d, of the cylindrical focus lens for compensation is given by:

$$d = -\frac{f_y}{f_c}\Delta$$

wherein $f_c$ is the focal length of the collimating lens 33, and $f_y$ is the focal length of the cylindrical focus lens.

In each of the above embodiments, the one or more characteristics of the optical system 20 that are adjusted in dependence on one or more of the optical properties of the cutting laser beam 18 detected by the imaging sensor 40 may be adjusted dynamically as the device 10 is moved over the skin 16 of a user, dependent on the specific generated information indicative of the presence of one or more objects representative of hair 17 and/or skin 16 in the cutting zone 15. An advantage of this arrangement is that the device is able to dynamically adjust operation of the optical system 20 to each part of a user's skin.

Although in the above described embodiments including adjustment of one or more optical components, adjustment of the or each optical component is achieved through adjustment of the orientation of the or each optical component, it will be understood that in an alternative embodiment adjustment of the or each optical component is achieved by having interchangeable optical components. In one embodiment, the or each actuator is operable to interchange two or more optical components.

It will be appreciated that the term "comprising" does not exclude other elements or steps and that the indefinite article "a" or "an" does not exclude a plurality.

The mere fact that certain measures are recited in mutually different dependent claims does not indicate that a combination of these measures cannot be used to an advantage. Any reference signs in the claims should not be construed as limiting the scope of the claims.

Although claims have been formulated in this application to particular combinations of features, it should be understood that the scope of the disclosure of the present invention also includes any novel features or any novel combinations of features disclosed herein either explicitly or implicitly or any generalisation thereof, whether or not it relates to the same invention as presently claimed in any claim and whether or not it mitigates any or all of the same technical problems as does the parent invention. The applicants hereby give notice that new claims may be formulated to such features and/or combinations of features during the prosecution of the present application or of any further application derived therefrom.

### Claims

1. A device (10) for cutting hair comprising
   a skin contacting face (13) that is arranged to be placed against a surface of the skin of a user during use,
   an optical system (20) configured to direct a cutting laser beam (18) across a cutting zone (15) parallel to and spaced from said skin contacting face to cut hairs extending into the cutting zone,
   a detector (40, 42) configured to generate information indicative of the presence of one or more objects representative of hair and/or skin in the cutting zone, and
   a controller (27) configured to determine the presence of one or more objects representative of hair and/or skin in the cutting zone and to adjust one or more characteristics of the optical system in dependence on the information generated by the detector, **characterised in that** the controller (27) is configured to generate information indicative of the distance between the one or more objects representative of hair and skin in the cutting zone (15) and the cutting laser beam (18) in dependence on information generated by the detector (40, 42), and to adjust the intensity of the cutting laser beam (18) in dependence on the information generated by the controller.

2. The device (10) according to claim 1, wherein the controller (27) is configured to reduce the intensity of the cutting laser beam (18) in dependence on the determination of the presence of one or more objects representative of skin in the cutting zone (15).

3. The device (10) according to claim 1 or claim 2, wherein the controller (27) is configured to increase the intensity of the cutting laser beam (18) in dependence on determination of the presence of one or more objects representative of hair in the cutting zone (15).

4. The device (10) according to any one of the preceding claims, wherein the controller (27) is additionally configured to adjust the path of the cutting laser beam (18).

5. The device (10) according to any preceding claim, wherein the detector (40) comprises an imaging sensor configured to generate information indicative of the presence of one or more objects representative of hair and/or skin received in a path of a light source (18) directed across the cutting zone (15).

6. A device (10) for cutting hair comprising
a skin contacting face (13) that is arranged to be placed against a surface of the skin of a user during use,
an optical system (20) configured to direct a cutting laser beam (18) across a cutting zone (15) parallel to and spaced from said skin contacting face to cut hairs extending into the cutting zone,
a detector (40) configured to generate information indicative of the presence of one or more objects representative of hair and/or skin in the cutting zone, and
a controller (27) configured to determine the presence of one or more objects representative of hair and/or skin in the cutting zone and to adjust one or more characteristics of the optical system in dependence on the information generated by the detector, **characterised in that** the controller (27) is configured to generate information indicative of the distance between the one or more objects representative of hair and skin in the cutting zone (15) and the cutting laser beam (18) in dependence on information generated by the detector (40, 42), and to adjust the path of the cutting laser beam (18) in dependence on the information generated by the controller; and **in that**
the detector (40) comprises an imaging sensor configured to generate information indicative of the presence of one or more objects representative of hair and/or skin received in a path of a light source (18) directed across the cutting zone (15).

7. The device (10) according to claim 5 or 6, wherein the light source (18) is a laser beam.

8. The device (10) according to claim 7, wherein the light source (18) is the cutting laser beam.

9. The device (10) according to claim 8, wherein the controller (27) is configured to generate information indicative of the distance between the cutting laser beam (18) and a reference position in dependence on information generated by the detector.

10. The device (10) according to any one of the preceding claims, wherein the detector (42) comprises a distance sensor configured to generate information indicative of the distance between one or more objects representative of hair and/or skin received in the cutting zone (15) and a reference position.

11. The device (10) according to any one of the preceding claims, wherein the controller (27) is configured to determine the presence of one or more objects representative of hair and skin in the cutting zone (15), and to disregard the presence of one or more objects representative of hair in the cutting zone when said one or more objects representative of hair in the cutting zone is determined.

12. The device (10) according to claim 4 or claim 6, or any of claims 6 to 11 when dependent on claim 4 or claim 6, wherein the controller (27) is configured to adjust the optical system (20) to modify the distance between the path of the cutting laser beam (18) and the skin contacting face (13).

13. A method of cutting hair comprising
directing a cutting laser beam (18) across a cutting zone (15) parallel to and spaced from said skin contacting face (13) to cut hairs extending into the cutting zone using an optical system (20),
generating information indicative of the presence of one or more objects representative of hair and/or skin in the cutting zone using a detector (40, 42),
determining the presence of one or more objects representative of hair and/or skin in the cutting zone and adjusting one or more characteristics of the optical system in dependence on the information generated by the detector, and
using a controller (27) to generate information indicative of the distance between the one or more objects representative of hair and skin in the cutting zone and the cutting laser beam in dependence on information generated by the detector and to adjust the intensity of the cutting laser beam (18) in dependence on the information generated by the controller.

14. A method of cutting hair comprising
directing a cutting laser beam (18) across a cutting zone (15) parallel to and spaced from said skin contacting face (13) to cut hairs extending into the cutting zone using an optical system (20),
generating information indicative of the presence of one or more objects representative of hair and/or skin in the cutting zone using a detector (40),
wherein the detector (40) comprises an imaging sensor configured to generate information indicative of the presence of one or more objects representative of hair and/or skin received in a path of a light source (18) directed across the cutting zone (15), determining the presence of one or more objects representative of hair and/or skin in the cutting zone and adjusting one or more characteristics of the optical system in dependence on the information generated by the detector, and

using a controller (27) to generate information indicative of the distance between the one or more objects representative of hair and skin in the cutting zone and the cutting laser beam in dependence on information generated by the detector and to adjust the path of the cutting laser beam (18) in dependence on the information generated by the controller.

15. A computer program comprising instructions which, when executed by at least one processor, cause the method of claim 13 or claim 14 to be performed.


**Patentansprüche**

1. Vorrichtung (10) zum Haareschneiden, umfassend eine Hautkontaktfläche (13), die angeordnet ist, während Verwendung gegen eine Fläche der Haut eines Anwenders angeordnet zu sein,

ein optisches System (20), das konfiguriert ist, einen Schneidelaserstrahl (18) über eine Schneidezone (15) parallel zu und von der Hautkontaktfläche beabstandet zu führen, um Haare zu schneiden, die sich in die Schneidezone erstrecken,

einen Detektor (40, 42), der konfiguriert ist, Informationen zu erzeugen, die die Gegenwart eines oder mehrerer Objekte anzeigen, die Haar und/oder Haut in der Schneidezone darstellen, und

eine Steuerung (27), die konfiguriert ist, die Gegenwart von einem oder mehr Objekten zu ermitteln, die Haar und/oder Haut in der Schneidezone darstellen, und ein oder mehrere Eigenschaften des optischen Systems abhängig von den durch den Detektor erzeugten Informationen einzustellen, **dadurch gekennzeichnet, dass**

die Steuerung (27) konfiguriert ist, Informationen zu erzeugen, die den Abstand zwischen dem einen oder den mehreren Objekten, die Haar und Haut in der Schneidezone (15) darstellen, und dem Schneidelaserstrahl (18) abhängig von durch den Detektor (40, 42) erzeugten Informationen angeben, und die Intensität des Schneidelaserstrahls (18) abhängig von den Informationen einzustellen, die durch die Steuerung erzeugt sind.

2. Vorrichtung (10) nach Anspruch 1, wobei die Steuerung (27) konfiguriert ist, die Intensität des Schneidelaserstrahls (18) abhängig von der Ermittlung der Gegenwart eines oder mehrerer Objekte, die Haut in der Schneidezone (15) darstellen, zu verringern.

3. Vorrichtung (10) nach Anspruch 1 oder Anspruch 2, wobei die Steuerung (27) konfiguriert ist, die Intensität des Schneidelaserstrahls (18) abhängig von einer Ermittlung der Gegenwart eines oder mehrerer Objekte, die Haar in der Schneidezone (15) darstellen, zu erhöhen.

4. Vorrichtung (10) nach einem der vorstehenden Ansprüche, wobei die Steuerung (27) zusätzlich konfiguriert ist, den Pfad des Schneidelaserstrahls (18) einzustellen.

5. Vorrichtung (10) nach einem der vorstehenden Ansprüche, wobei der Detektor (40) einen Bildgebungssensor umfasst, der konfiguriert ist, Informationen zu erzeugen, die die Gegenwart eines oder mehrerer Objekte anzeigen, die Haar und/oder Haut darstellen, die in einem Pfad einer Lichtquelle (18) empfangen werden, der über die Schneidezone (15) gerichtet ist.

6. Vorrichtung (10) zum Haareschneiden, umfassend eine Hautkontaktfläche (13), die angeordnet ist, während Verwendung gegen eine Fläche der Haut eines Anwenders platziert zu werden,

ein optisches System (20), das konfiguriert ist, einen Schneidelaserstrahl (18) über eine Schneidezone (15) parallel zu und beanstandet von der Hautkontaktfläche zu führen, um Haare zu schneiden, die sich in die Schneidezone erstrecken,

einen Detektor (40), der konfiguriert ist, Informationen zu erzeugen, die die Gegenwart von einem oder mehreren Objekten anzeigen, die Haar und/oder Haut in der Schneidezone darstellen, und

eine Steuerung (27), die konfiguriert ist, die Gegenwart von einem oder mehreren Objekten zu ermitteln, die Haar und/oder Haut in der Schneidezone darstellen, und eine oder mehrere Eigenschaften des optischen Systems abhängig von den durch den Detektor erzeugten Informationen einzustellen, **dadurch gekennzeichnet, dass**

die Steuerung (27) konfiguriert ist, Informationen zu erzeugen, die den Abstand zwischen dem einen oder den mehreren Objekten, die Haar und Haut in der Schneidezone (15) darstellen, und dem Schneidelaserstrahl (18) abhängig von durch den Detektor (40, 42) erzeugten Informationen anzuzeigen und den Pfad des Schneidelaserstrahls (18) abhängig von den durch die Steuerung erzeugten Informationen einzustellen; und dadurch, dass

der Detektor (40) einen Bildgebungssensor umfasst, der konfiguriert ist, Informationen zu erzeugen, die die Gegenwart eines oder mehrerer Objekte anzeigen, die Haar und/oder Haut darstellen, die in einem Pfad einer Lichtquelle (18) empfangen werden, der über die Schneidezone (15) gerichtet ist.

7. Vorrichtung (10) nach Anspruch 5 oder 6, wobei die Lichtquelle (18) ein Laserstrahl ist.

8. Vorrichtung (10) nach Anspruch 7, wobei die Lichtquelle (18) der Schneidelaserstrahl ist.

9. Vorrichtung (10) nach Anspruch 8, wobei die Steuerung (27) konfiguriert ist, Informationen zu erzeugen, die den Abstand zwischen dem Schneidelaserstrahl (18) und einer Referenzposition abhängig von Informationen anzeigen, die durch den Detektor erzeugt sind.

10. Vorrichtung (10) nach einem der vorstehenden Ansprüche, wobei der Detektor (42) einen Abstandssensor umfasst, der konfiguriert ist, Informationen zu erzeugen, die den Abstand zwischen einem oder mehreren Objekten, die Haar und/oder Haut darstellen, die in der Schneidezone (15) empfangen werden, und einer Referenzposition anzeigen.

11. Vorrichtung (10) nach einem der vorstehenden Ansprüche, wobei die Steuerung (27) konfiguriert ist, die Gegenwart von einem oder mehreren Objekten zu ermitteln, die Haar und Haut in der Schneidezone (15) darstellen, und die Gegenwart von einem oder mehreren Objekten, die Haart in der Schneidezone darstellen, unbeachtet zu lassen, wenn das eine oder die mehreren Objekte, die Haar in der Schneidezone darstellen, ermittelt sind.

12. Vorrichtung (10) nach Anspruch 4 oder Anspruch 6, oder irgendeinem der Ansprüche 6 bis 11, wenn abhängig von Anspruch 4 oder 6, wobei die Steuerung (27) konfiguriert ist, das optische System (20) einzustellen, den Abstand zwischen dem Pfad des Schneidelaserstrahls (18) und der Hautkontaktfläche (13) zu modifizieren.

13. Verfahren zum Haareschneiden, umfassend Richten eines Schneidelaserstrahls (18) über eine Schneidezone (15) parallel zu und beabstandet von der Hautkontaktfläche (13), um Haare, die sich in die Schneidezone erstrecken, unter Verwendung eines optischen Systems (20) zu schneiden,
Erzeugen von Informationen, die die Gegenwart von einem oder mehreren Objekten anzeigen, die Haar und/oder Haut in der Schneidezone darstellen, unter Verwendung eines Detektors (40, 42),
Ermitteln der Gegenwart eines oder mehrerer Objekte, die Haar und/oder Haut in der Schneidezone darstellen
und Einstellen einer oder mehrere Eigenschaften des optischen Systems, abhängig von den Informationen, die durch den Detektor erzeugt sind, und
Verwenden einer Steuerung (27), um Informationen zu erzeugen, die den Abstand zwischen dem einen oder den mehreren Objekten, die Haar und Haut in der Schneidezone darstellen, und dem Schneidelaserstrahl abhängig von Informationen, die durch den Detektor erzeugt sind, anzeigen, und die Intensität des Schneidelaserstrahls (18) abhängig von den Informationen, die durch die Steuerung erzeugt sind, einzustellen.

14. Verfahren zum Haareschneiden, umfassend Führen eines Schneidelaserstrahls (18) über eine Schneidezone (15) parallel zu und beabstandet von der Hautkontaktfläche (13), um Haare, die sich in die Schneidezone erstrecken, unter Verwendung eines optischen Systems (20) zu schneiden,
Erzeugen von Informationen, die die Gegenwart eines oder mehrerer Objekte anzeigen, die Haar und/oder Haut in der Schneidezone darstellen, unter Verwendung eines Detektors (40),
wobei der Detektor (40) einen Bildgebungssensor umfasst, der konfiguriert ist, Informationen zu erzeugen, die die Gegenwart von einem oder mehreren Objekten anzeigen, die Haar und/oder Haut darstellen, die in einem Pfad einer Lichtquelle (18) aufgenommen sind, der über die Schneidezone (15) führt,
Ermitteln der Gegenwart eines oder mehrerer Objekte, die Haar und/oder Haut in der Schneidezone darstellen
und Einstellen einer oder mehrerer Eigenschaften des optischen Systems abhängig von den Informationen, die durch den Detektor erzeugt sind, und
Verwenden einer Steuerung (27), um Informationen zu erzeugen, die den Abstand zwischen dem einen oder den mehreren Objekten, die Haar und Haut in der Schneidezone darstellen, und dem Schneidelaserstrahl abhängig von Informationen, die durch den Detektor erzeugt sind, anzeigen und den Pfad des Schneidelaserstrahls (18) abhängig von den Informationen einzustellen, die durch die Steuerung erzeugt sind.

15. Computerprogramm, umfassend Anweisungen, die, wenn durch mindestens einen Prozessor durchgeführt, veranlassen, dass das Verfahren nach Anspruch 13 oder Anspruch 14 ausgeführt wird.

**Revendications**

1. Dispositif (10) pour couper les cheveux comprenant une face entrant en contact avec la peau (13) qui est agencée pour être placée contre une surface de la peau d'un utilisateur pendant l'utilisation,
un système optique (20) configuré pour diriger un faisceau laser de coupe (18) d'un bout à l'autre d'une zone de coupe (15) parallèle à et espacé de ladite face entrant en contact avec la peau pour couper les cheveux s'étendant dans la zone de coupe,
un détecteur (40, 42) configuré pour produire des informations indicatives de la présence d'un ou plusieurs objets représentatifs de cheveux et/ou de peau dans la zone de coupe, et
une unité de commande (27) configurée pour déterminer la présence d'un ou plusieurs objets représentatifs de cheveux et/ou de peau dans la zone de coupe et pour ajuster une ou plusieurs caractéristiques du système optique en fonction des informations produites par le détecteur,

**caractérisé en ce que** l'unité de commande (27) est configurée pour produire des informations indicatives de la distance entre le ou les plusieurs objets représentatifs de cheveux et de peau dans la zone de coupe (15) et le faisceau laser de coupe (18) en fonction des informations produites par le détecteur (40, 42), et pour ajuster l'intensité du faisceau laser de coupe (18) en fonction des informations produites par l'unité de commande.

2. Dispositif (10) selon la revendication 1, dans lequel l'unité de commande (27) est configurée pour réduire l'intensité du faisceau laser de coupe (18) en fonction de la détermination de la présence d'un ou plusieurs objets représentatifs de peau dans la zone de coupe (15).

3. Dispositif (10) selon la revendication 1 ou la revendication 2, dans lequel l'unité de commande (27) est configurée pour augmenter l'intensité du faisceau laser de coupe (18) en fonction de la détermination de la présence d'un ou plusieurs objets représentatifs de cheveux dans la zone de coupe (15).

4. Dispositif (10) selon l'une quelconque des revendications précédentes, dans lequel l'unité de commande (27) est de plus configurée pour ajuster le chemin du faisceau laser de coupe (18).

5. Dispositif (10) selon l'une quelconque des revendications précédentes, dans lequel le détecteur (40) comprend un capteur d'imagerie configuré pour produire des informations indicatives de la présence d'un ou plusieurs objets représentatifs de cheveux et/ou de peau reçus dans un chemin d'une source de lumière (18) dirigée d'un bout à l'autre de la zone de coupe (15).

6. Dispositif (10) pour couper les cheveux comprenant une face entrant en contact avec la peau (13) qui est agencée pour être placée contre une surface de la peau d'un utilisateur pendant l'utilisation, un système optique (20) configuré pour diriger un faisceau laser de coupe (18) d'un bout à l'autre d'une zone de coupe (15) parallèle à et espacé de ladite face entrant en contact avec la peau pour couper les cheveux s'étendant dans la zone de coupe, un détecteur (40) configuré pour produire des informations indicatives de la présence d'un ou plusieurs objets représentatifs de cheveux et/ou de peau dans la zone de coupe, et une unité de commande (27) configurée pour déterminer la présence d'un ou plusieurs objets représentatifs de cheveux et/ou de peau dans la zone de coupe et pour ajuster une ou plusieurs caractéristiques du système optique en fonction des informations produites par le détecteur,

**caractérisé en ce que** l'unité de commande (27) est configurée pour produire des informations indicatives de la distance entre le ou les plusieurs objets représentatifs de cheveux et de peau dans la zone de coupe (15) et le faisceau laser de coupe (18) en fonction d'informations produites par le détecteur (40, 42), et pour ajuster le chemin du faisceau laser de coupe (18) en fonction des informations produites par l'unité de commande ; et **en ce que** le détecteur (40) comprend un capteur d'imagerie configuré pour produire des informations indicatives de la présence d'un ou plusieurs objets représentatifs de cheveux et/ou de peau reçus dans un chemin d'une source de lumière (18) dirigée d'un bout à l'autre de la zone de coupe (15).

7. Dispositif (10) selon la revendication 5 ou 6, dans lequel la source de lumière (18) est un faisceau laser.

8. Dispositif (10) selon la revendication 7, dans lequel la source de lumière (18) est le faisceau laser de coupe.

9. Dispositif (10) selon la revendication 8, dans lequel l'unité de commande (27) est configurée pour produire des informations indicatives de la distance entre le faisceau laser de coupe (18) et une position de référence en fonction d'informations produites par le détecteur.

10. Dispositif (10) selon l'une quelconque des revendications précédentes, dans lequel le détecteur (42) comprend un capteur de distance configuré pour produire des informations indicatives de la distance entre un ou plusieurs objets représentatifs de cheveux et/ou de peau reçus dans la zone de coupe (15) et une position de référence.

11. Dispositif (10) selon l'une quelconque des revendications précédentes, dans lequel l'unité de commande (27) est configurée pour déterminer la présence d'un ou plusieurs objets représentatifs de cheveux et de peau dans la zone de coupe (15), et pour ignorer la présence d'un ou plusieurs objets représentatifs de cheveux dans la zone de coupe lorsque lesdits un ou plusieurs objets représentatifs de cheveux dans la zone de coupe sont déterminés.

12. Dispositif (10) selon la revendication 4 ou la revendication 6, ou l'une quelconque des revendications 6 à 11 lorsque dépendante de la revendication 4 ou de la revendication 6, dans lequel l'unité de commande (27) est configurée pour ajuster le système optique (20) pour modifier la distance entre le chemin du faisceau laser de coupe (18) et la face entrant en contact avec la peau (13).

13. Procédé de coupe de cheveux comprenant

la direction d'un faisceau laser de coupe (18) d'un bout à l'autre d'une zone de coupe (15) parallèle à et espacé de ladite face entrant en contact avec la peau (13) pour couper les cheveux s'étendant dans la zone de coupe en utilisant un système optique (20),

la production d'informations indicatives de la présence d'un ou plusieurs objets représentatifs de cheveux et/ou de peau dans la zone de coupe en utilisant un détecteur (40, 42),

la détermination de la présence d'un ou plusieurs objets représentatifs de cheveux et/ou de peau dans la zone de coupe

et l'ajustement d'une ou plusieurs caractéristiques du système optique en fonction des informations produites par le détecteur, et

l'utilisation d'une unité de commande (27) pour produire des informations indicatives de la distance entre le ou les plusieurs objets représentatifs de cheveux et de peau dans la zone de coupe et le faisceau laser de coupe en fonction d'informations produites par le détecteur et pour ajuster l'intensité du faisceau laser de coupe (18) en fonction des informations produites par l'unité de commande.

14. Procédé de coupe de cheveux comprenant

la direction d'un faisceau laser de coupe (18) d'un bout à l'autre d'une zone de coupe (15) parallèle à et espacé de ladite face entrant en contact avec la peau (13) pour couper les cheveux s'étendant dans la zone de coupe en utilisant un système optique (20),

la production d'informations indicatives de la présence d'un ou plusieurs objets représentatifs de cheveux et/ou de peau dans la zone de coupe en utilisant un détecteur (40),

dans lequel le détecteur (40) comprend un capteur d'imagerie configuré pour produire des informations indicatives de la présence d'un ou plusieurs objets représentatifs de cheveux et/ou de peau reçus dans un chemin d'une source de lumière (18) dirigée d'un bout à l'autre de la zone de coupe (15),

la détermination de la présence d'un ou plusieurs objets représentatifs de cheveux et/ou de peau dans la zone de coupe

et l'ajustement d'une ou plusieurs caractéristiques du système optique en fonction des informations produites par le détecteur, et

l'utilisation d'une unité de commande (27) pour produire des informations indicatives de la distance entre le ou les plusieurs objets représentatifs de cheveux et de peau dans la zone de coupe et le faisceau laser de coupe en fonction d'informations produites par le détecteur et pour ajuster le chemin du faisceau laser de coupe (18) en fonction des informations produites par l'unité de commande.

15. Programme informatique comprenant des instructions qui, lorsqu'elles sont exécutées par au moins un processeur, amènent le procédé selon la revendication 13 ou la revendication 14 à être effectué.

EP 3 171 806 B1

FIG. 1

FIG. 2

*10*

*FIG. 3*

*50*  18

*52*  18

17

17

16

17

*FIG. 4*                *FIG. 5*

600

601

602    No

Yes

603

604    Yes

No

605

*FIG. 6*

FIG. 9

FIG. 10

FIG. 11

FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 199216338 A
- US 5993440 A

- WO 2013175355 A1